# EUROPEAN PATENT APPLICATION

(11) **EP 4 064 729 A1**
(43) Date of publication of application: **28.09.2022**
(21) Application number: 21163914.1
(22) Date of filing: 22.03.2021
(51) Int. Cl.: H04R 25/00, A61B 5/11, A61B 5/00

(54) **DETECTING AND QUANTIFYING A LIQUID AND/OR FOOD INTAKE OF A USER WEARING A HEARING DEVICE**

(71) Applicant: Sonova AG, 8712 Stäfa (CH)
(72) Inventor: FEILNER, Manuela, 8132 Egg (CH)
(74) Representative: Qip Patentanwälte Dr. Kuehn & Partner mbB

(57) **Abstract**

A method for detecting and quantifying a liquid and/or food intake of a user wearing a hearing device (12) which comprises at least one microphone (20). The method comprises: receiving an audio signal from the at least one microphone (20) and/or a sensor signal from at least one further sensor (50); collecting and analyzing the received audio signal and/or further sensor signals so as to detect each time the user drinks and/or eats something, wherein drinking is distinguished from eating, and so as to determine values indicative of how often this is detected and/or a respective amount of liquid and/or food ingested by the user; wherein the step of analyzing includes applying one or more machine learning algorithms in the hearing device (12) or in a hearing system (10), part of which the hearing device (12) is, or in a remote server or cloud connected to it. The method further comprises storing the determined values in the hearing system (10) and, based on the stored values, generating a predetermined type of output.

## Description

### FIELD OF THE INVENTION

The invention relates to a method, a computer program and a computer-readable medium for detecting and quantifying a liquid and/or food intake of a user wearing a hearing device which comprises at least one microphone. Furthermore, the invention relates to a hearing system which comprises at least one hearing device of this kind and optionally a connected user device, such as a smartphone.

### BACKGROUND OF THE INVENTION

Hearing devices are generally small and complex devices. Hearing devices can include a processor, microphone, an integrated loudspeaker as a sound output device, memory, housing, and other electronical and mechanical components. Some example hearing devices are Behind-The-Ear (BTE), Receiver-In-Canal (RIC), In-The-Ear (ITE), Completely-In-Canal (CIC), and Invisible-In-The-Canal (IIC) devices. A user can prefer one of these hearing devices compared to another device based on hearing loss, aesthetic preferences, lifestyle needs, and budget.

Many of the elderly often do not drink sufficiently, because of several reasons, e. g. lack of thirst, lack of remembering that they shall drink. Subsequently, they have a high risk to dehydrate, which might cause problems such as dizziness, a condition of mental decline etc.

Hearing devices can be used to monitor the drinking behavior of their users and to counsel the users by helping them to develop a healthy hydrated lifestyle. To this end, the hearing system shall detect automatically the fluid intake of a user.

In this context, for example, DE 10 2018 204 695 A1 discloses using hearing systems, which comprise hearing devices or other devices worn by the user on his head, such as earphones or headsets, for health monitoring. The document proposes recognizing a large variety of specific symptoms or irregularities in the breathing, speaking, sleeping, walking, chewing, swallowing or other body functions of the user by means of sensors such as microphones, vibration sensors or accelerometers e. g. integrated in the hearing device worn by the user. Use cases address symptoms of various diseases such as Parkinson, traumatic brain injury, tics, bruxism, hiccup, allergic reactions, coughing fits and many more. Inter alia, swallowing is proposed to be recognized by sensing a typical sound moving downwards away from the hearing device with a microphone, in combination with multiple characteristic muscle contractions sensed by a vibration sensor in the ear canal. It is also mentioned that a single gulp may be interpreted as swallowing saliva, but possibly also as medication intake, which may be used to monitor the latter. It is also briefly mentioned that a drinking process might be detected as more or less regular gulps by a microphone or a vibration sensor, and that the user may also be reminded to drink. However, no detailed evaluation algorithms are described for these different use cases.

A specific method of detecting a dehydration by measuring a water level in the brain of the hearing device users, or a bio impedance, is disclosed in US 2017/0289704 A1, based on the hypothesis that the magnetic/electric conductance in the head varies with the relative water level in the head, at least on a short term. If the measured water level is below a predefined threshold, a reminder signal reminding the user to drink something is generated by at least one of the hearing devices and sent to the user via e.g. a smartphone or as a direct audio reminder, e.g. a speech message.

Another problem known in the art is that the elderly often lose their appetite, whereas bad eating habits may increase obesity or the risk of heart diseases.

### DESCRIPTION OF THE INVENTION

It is an objective of the invention to provide a reliable and robust method and system for detecting and quantifying a liquid and/or food intake of a user wearing a hearing device. It is a further objective of the invention to provide a reliable and robust method of detecting dehydration and/or monitoring a correct intake of medication. It is a further objective of the invention to enhance the user's experience of drinking or, respectively, eating by augmented reality means.

These objectives are achieved by the subject-matter of the independent claims. Further exemplary embodiments are evident from the dependent claims and the following description.

A first aspect of the invention relates to a method for detecting and quantifying a liquid and/or food intake of a user wearing a hearing device which comprises at least one microphone.

The method may be a computer-implemented method, which may be performed automatically in the hearing device and/or in another device of a hearing system, part of which the user's hearing device is. As described in more detail herein below, the hearing system may consist of the hearing device alone, or may be a binaural hearing system comprising two hearing devices worn by the same user, or may comprise the hearing device and a remote device portable by the user, such as a smartphone or smartwatch, connected to the hearing device. One or both of the hearing devices may be worn on and/or in an ear of the user. A hearing device may be a hearing aid, which may be adapted for compensating a hearing loss of the user. Also a cochlear implant may be a hearing device.

According to an embodiment of the invention, the method comprises receiving an audio signal from the at least one microphone and/or a sensor signal from at least one further sensor. As described in more detail herein below, the further sensors may include any types of physical or physiological sensors - e.g. movement sensors, such as accelerometers, and/or optical sensors, such as cameras, and/or temperature sensors and/or heart rate sensors etc. - integrated in the hearing device or possibly also in a connected user device, such as a smartphone or a smartwatch.

According to an embodiment of the invention, the method further comprises collecting and analyzing the received audio signal and/or further sensor signals so as to detect each time the user drinks and/or eats something, wherein drinking is distinguished from eating. The analysis further includes determining values indicative of how often this is detected and/or a respective amount of liquid and/or food ingested by the user.

The determined values are then stored in the hearing system and, based on the stored values, a predetermined type of output is generated, in particular, to the user and/or to a third party, such as a person close to the user and/or a health care professional. The predetermined type of output may, for example, include one or more of the following: a textual, acoustical, graphical, vibrational output, as well as an output generated using augmented reality and/or an interactive user interface etc. The specific choice of a suitable output may, for instance, depend on the specific goals and use cases addressed in the different embodiments of the invention as described herein below.

According to an embodiment of the invention, the above-mentioned step of analyzing includes applying one or more machine learning algorithms, subsequently and/or in parallel, in the hearing device or in the hearing system or in a remote server or cloud connected to the hearing device or system. By applying a suitable machine learning algorithm, a most reliable and/or robust method of detecting and quantifying the liquid and/or food intake of the user may be provided.

This may, for example, be achieved due to the capability of a machine learning algorithm to automatically adapt the analysis to individual drinking and/or eating habits of the user, which may also take into account that habits of one and the same user are often changing with time, e.g. vary from season to season or may even rapidly change with a changing life style when the user moves to another country, changes his job, marries etc. Furthermore, the reliability and robustness may also be increased by a machine learning algorithm which is configured to automatically supplement the analysis by adding new sensor-based recognition criteria of the user's drinking and eating behavior, after having started with some basic or initial recognition criteria. In the course of this, for example, sensor signals of additional sensors provided in the hearing system may be automatically included in the analysis algorithm by machine learning.

Therefore, according to an embodiment of the invention, at least one of the machine learning algorithms may be applied in its training phase so as to learn user-specific manners of drinking and/or eating, in order to reliably detect whenever the user drinks or eats something and to distinguish eating from drinking. For example, the machine learning algorithms may be applied to learn that - or how exactly - the user typically elevates his head slightly to take a gulp from a bottle or a sip from a glass or cup of liquid; or to learn that - or how exactly - the user is typically lowering his head when taking a bite when eating. The newly learned user-specific manners are then incorporated in the future analysis step. Further details and examples are described herein below.

Any method features described herein with respect to a drinking process (liquid intake) may also be applied by analogy to detecting and quantifying an eating process (food intake) of the hearing device user. The detection algorithm is then correspondingy adapted to use and interpret the collected sensor signals so as to distinguish eating from drinking and so as to learn the respective specific recognition and quantification criteria.

Particularly important examples of respective output generation in either case are described in more detail herein below. They provide, for example, novel and/or improved methods of dehydration detection, verification of a medication intake, enhancement of a liquid intake desire and/or enhancement of an eating or drinking experience of the hearing device user, so as to raise the user's life quality and support a healthy living style.

According to an embodiment of the invention, in the step of analyzing, two or more different (in particular, subsequent) phases of drinking or, respectively, eating, are distinguished in the course of detecting a liquid and/or food intake of the user. Thereby, again, the robustness and reliability of the method may be increased.

Since different sensors might be better suited to detect the different phases, the analysis of the different phases may be based on signals from at least partly different sensors or groups of sensors. On the other hand, the analysis of different phases may also be performed by at least partly different machine learning algorithms. In the case of subsequent phases, the respective algorithms may also be applied in a corresponding subsequent manner.

In this embodiment, the different phases of drinking may, for example, comprise one or more of the following phases (whereas the different phases of eating may be defined in a similar manner by analogy):
A first phase of drinking may be bringing a source of liquid in contact with the mouth. This may, for example, be grabbing a glass or bottle or another vessel containing the liquid. Alternatively, this may also be lowering the head so as to reach a jet of water, e. g. emanating from a water tap, with the mouth. Detecting this phase may be based, inter alia, on a signal from at least one movement sensor sensing a corresponding movement of some upper body part of the user. Suitable movement sensors may include movement sensors, e.g. accelerometers, provided at the user's head, e.g. in the hearing device, and/or at the user's wrist, e.g. in a wrist band, and/or at another upper body part of the user.
A second phase of drinking may be gulping or sipping the liquid. Detecting this phase may be based, inter alia, on a signal from the at least one microphone, e.g. in a hearing device, and/or on a signal from at least one movement sensor sensing a corresponding movement of the user's throat, head and/or breast. Suitable movement sensors may include at least one accelerometer provided in the hearing device and/or on a head, neck or breast of the user.
A third phase of drinking may be removing the mouth from the source of liquid. This may be, for examlpe, putting the glass, bottle or another vessel back on the table. Alternatively, this may also be raising the head away from the jet of water. Detecting this phase may be based, inter alia, on a signal from the at least one microphone and/or on a signal from at least one movement sensor sensing a corresponding movement of some upper body part of the user. Suitable movement sensors may include movement sensors, such as accelerometers, provided at the user's head, e.g. in the hearing device, and/or at the user's wrist, e.g. in a wrist band, and/or at another upper body part of the user.

Correspondingly, the detection algorithm might also consist of three parts, which are processed in a timely sequence, according to the described three drinking phases. Furthermore, instead of the above-mentioned exemplary three different phases, another number and/or types of different phases may be identifiable in the course of detecting a drinking or eating process of the user, for example two or four subsequent phases.

According to an embodiment of the invention, at least one of the machine learning algorithms applied in the analysis step is based on an artificial neural network. The input data set for the neural network is provided at a respective time point by the sensor data collected over a predetermined period of time up to this time point. The output data set for the respective time point includes a frequency or number of detected liquid and/or food intakes as well as a respective or an overall amount of the liquid and/or food ingested by the user and/or a duration of the detected liquid and/or food intakes. The artificial neural network may, for example, be a deep neural network including one or multiple hidden layers.

The training phase of the (deep) neural network may be implemented by a supervised learning, in which the algorithm is trained using a database of labeled input sensor data with corresponding output data sets. A suitable database of training data, working well for the above-described analysis, contains, for example, recorded swallowing or, respectively, gulping sounds of a large number of people - in this example approximately 1000 adults. Alternatively or additionally, representative sound recordings of drinking, swallowing, gulping, chewing people available in the Internet may also be used as training data.

Furthermore, the training phase may be implemented by an unsupervised learning in an environment with still more information available (Internet of Things), e.g. by using data transmitted from smart devices related to drinking and eating, such as smart water bottles known in the art, and/or additional information within a smart home and/or input from the user via an interactive user interface as described herein below. The training phase may also comprise reinforcement learning or deep reinforcement learning.

Moreover, the detection and/or quantification algorithm mentioned herein above and below might be a (deep) neural network, a statistical approach known as a multivariate analysis of variance (Manova), Support Vector Machines (SVM) or any other machine learning algorithm, pattern recognition algorithm or statistical approach. The detection algorithm might consist of three parts, which are processed in a timely sequence, according to the three drinking phases described above. This is, however, not necessary. Instead of processing the three phases in a temporal manner and separately, the drinking (and, by analogy, eating) procedure might also be detected without distinguishing any phases. A deep learning algorithm might find other criteria during a training phase.

For instance, instead of distinguishing the above-described three (or any other number of) phases of drinking a priori in the step of analyzing, a temporal dynamic behavior of the drinking process (in other words, the "history" of the drinking process), may be incorporated by applying a Hidden Markow Model (HMM) or a recurrent neural network (RNN), such as a long short-term memory (LSTM) or a gated recurrent unit (GRU).

An artificial neural network or other machine learning algorithms as described herein may be implemented directly in the hearing device, e.g. in a chip with a high degree of parallel processing, being integrated in the hearing device to this end. Alternatively or additionally, this may also be implemented in any other device of the hearing system. Since the analysis steps of drinking or eating detection and quantification, or further items such as dehydration detection described herein above and below, must not necessarily be solved in real time and must not necessarily be all processed within the hearing device itself, diverse and more complex algorithms and processes than in the cited prior art are implementable.

In any of the embodiments described herein, the hearing device or system may be configured to combine and analyse signals of several sensors from the following exemplary list of sensors. Corresponding detection criteria (as mentioned for each sensor) are, in general, user-specific and/or use-case specific. This may be learned by a suitable machine learning algorithm, as described herein above and in the following. A combination of several sensors may, for instance, be suitable to detect all the above-mentioned phases of the drinking (or eating) procedure:
- Accelerometers (or gyroscopes) in the hearing device may be used to detect when the user elevates his head slightly to take a sip. In contrast, to detect eating, the detection algorithm may be based on the fact that the user is rather lowering his head when taking a bite.
- In addition, the user might wear a smartwatch or a wristband around his wrist with integrated movement sensors. While the user brings the glass to his mouth and while drinking, his arm follows a specific gesture, which may be recognized by these sensors so as to indicate the possible action of drinking. This movement pattern might also be used to distinguish between drinking, eating and other possible actions of the user.
- In addition, a drinking bottle might incorporate sensors and be connected to the smart home (also known as smart water bottles). The hearing device or system may be connected to the smart home as well and receive those signals to incorporate them into the detection process. Alternatively, those data may be collected in a cloud or a smart home device to compute the algorithms.
- Heart rate sensors might be incorporated to detect intake of liquid or food: the pulse of the heart can be influenced and altered by swallowing liquid or solid nutrition. This effect may also strongly vary depending on the type and quantity of liquid or food taken by the user, which might be incorporated in the analysis algorithm to determine the respective values or detect the difference.
- Other sensors, such as electroencephalographic (EEG) sensors, eye gaze or a camera within the smart home or integrated into glasses might be incorporated for detection.

In addition, other sensors might be applied, such as:
- Acoustic sensors: the microphones within the hearing device or microphones in the ear canal might detect the gulping sound or might improve the detection rate by recognizing and/or excluding other actions, such as own-voice or eating.
- Voice Pickup sensors (VPUs) might be used to improve the detection of gulping and distinguish it from eating, and vice versa.

According to an embodiment of the invention, in the step of analyzing, a dehydration risk of the hearing device user is estimated depending on the determined values of the amount and of a frequency (i. e. of how often he has drunk) of the user's liquid intake. The risk of dehydration may be estimated depending on the drinking behavior, e.g. the determined amount of liquid intake is compared with a predetermined value "Dmin" which describes the minimum amount of drinking the user needs to take. This value can be a fixed number or might be calculated depending on the environmental conditions, such as the ambient temperature, on the activity behavior of the user, e.g. walking or sitting, and/or on medical conditions, such as weight etc. The value "Dmin" can be adapted by the user via the interactive user interface described below and/or by his doctor.

In this embodiment, the generated output may be configured depending on the estimated dehydration risk so as to counsel the user to ingest a lacking amount of liquid and/or so as to inform the user and/or a person close to the user and/or a health care professional about the estimated dehydration risk. Counselling the user may be implemented e.g. using a smartphone app and/or acoustically via an integrated loudspeaker of the hearing device and/or via vibration on devices such as wristband, smartwatch, and/or via augmented reality using the user's glasses to generate a virtual image of a glass or bottle.

Alternatively, instead of a dehydration system design where users are admonished to drink more, the system design may enhance the desire to drink when dehydration is detected. This type of output depending on the estimated dehydration risk is described in more detail further below.

According to an embodiment of the invention, an interactive user interface is provided in the hearing system and the steps of analyzing and/or generating an output are supplemented by an interaction with the user via the interactive user interface, wherein the user is enabled to input additional information pertaining to his liquid and/or food intake.

For example, such an interface may be configured such as to enable the user to manually enter the data for drinking behaviour and/or to correct and/or specify the liquid intake. It might further provide the user with a possibility to add comments about subjective descriptions and ratings about his health state, mental condition, feelings, thirst, appetite, social condition (e.g. alone or with company), cause of fluid intake, intake of medication, etc. Thereby, for example, the hearing system might ask the user proactively whether he has taken medication right after the system has detected fluid intake. The proactive questioning might be during a specific time predefined and stored in the hearing system, such as the morning, the evening, just before or after taking a meal, which, in turn, might be determined using the analysis algorithms described herein. This proactive question shall help the user to keep track about the intake of medication.

The interactive user interface might also help to improve the accuracy of the detection in general or within a learning phase of the analysis algorithm. It may also serve for a more elaborated therapy.

The interactive user interface might comprise one or more of the following types of user interface:
- an app on a smartphone, smartwatch or another connected user device, which provides a correction mode showing e.g. the detected gulpes right after detection.
- An entry mode provided in the hearing device (e.g. by voice recognition or via a button) or on a connected user device, configured to enable the user to specify the liquid and/or food intake.
- A conversational user interface incorporated in the hearing device(s).

According to an embodiment of the invention, additional information about a need to take a predetermined medication (e. g. on a regular basis) is stored in the hearing system. In this embodiment, when a fluid intake of the user is detected, the output is generated depending on this additional information and comprises questioning the user, via the interactive user interface, whether he has taken the predetermined medication. The user's response to this question via the interactive user interface may then be, for example, stored in the hearing system and/or transmitted to the user and/or to a third person close to the user and/or a health care professional, so as to verify that the user has taken the predetermined medication.

Alternatively or additionaly to questioning the user, detecting the intake of medication may also be based on the fluid intake detection in combination with the analysis of head movements of the user. This may also be implemented by the sensor-based machine learning algorithm as described herein above and below.

According to an embodiment of the invention, in the step of generating an output depending on the determined frequency and amount of the liquid or, respectively, food ingested by the user, an output configured such as to enhance the user's desire to drink or, respectively, eat something is generated by augmented reality means in the hearing system. In particular, this may be an output generated upon estimating a dehydration risk in the embodiment described further above, as alternative to an output which admonishes the user to drink more.

In this embodiment, an output configured to enhance the user's desire to drink or eat something may, for example, be reached with suitable sound, smell and video effects and methods. This may include augmenting specific sounds or music associated by the user with drinking or suitable to enhance a drinking experience. This may also be achieved by presenting specific sounds or music associated by the user with eating and/or which can lead to an enhanced tasting-experience. The suitable effects may be very user-specific and be determined as additional information by machine learning in the analysis step of the present method, and/or indicated by the user via the above-described interactive user interface.

The sounds or optical effects to be presented in order to enhance the user's desire to drink might, for example, strongly differ dependent on the drink to be taken and on the user's cultural background and music preference, which may be evaluated by the hearing system in the analysis step. Same also applies to flavor effects. Furthermore, it is also known that there may be a strong user-specific correlation between smell and sound, for instance bound to certain situations in life remembered by the user, so that a suitable combination of smell and sound may lead to a still stronger desire of the user to drink or, respectively, eat something special in this embodiment. All this also applies to video in addition to or instead of sound effects.

Specifically, one or more of the following outputs may be generated by the hearing system in this embodiment:
▪ An already existing drinking glass or bottle in the field of vision of the user might be augmented visually with augmenting colors representing the fluid, e.g. using smart glasses connected to the hearing device,
▪ the hearing system might display sounds, such as water drops, sound which represents filling a glass with water or the splashing sound of opening a beer, etc., which might augment the desire to drink.
▪ For elderly, where all senses decline, the enhancement of smell might increase the experience as well. Depending on what the user is drinking, the smart home connected to the hearing device might pump smell into the room according to the drink. E.g. when the content of the bottle or glass next to the user consists of tea, the smell consists of a strong tea smell, when the glass contains orange juice, the smell consists of fresh oranges.
▪ Also a video might be played representing a drinking experience and the corresponding sound. If the user is watching TV (on a TV set connected to the hearing device) for a long time, videos without the commercial content can be shown in between to enhance the user's desire to drink or eat something needed for the user's health.

According to an embodiment of the invention, when detecting that the user is drinking or, respectively, eating something and/or upon detecting which kind of liquid or, respectively, food the user is taking, an output configured such as to enhance the user's experience of drinking or, respectively, eating is generated by augmented reality means in the hearing system depending on this (in particular multisensory) detection. This shall be reached with similar methods as described for the previous embodiment: with sound, smell and video. Furthermore, a suitable output may be determined in a similar user-specific manner as described above.

Furthermore, a similar algorithm as described herein can be applied to monitor any other health related activities and body symptoms, such as e.g. mentioned at the outset.

The above-described embodiments, where the output is generated such as to enhance the user's desire to ingest some healthy liquid or food or to enhance the respective eating or drinking experience, might be particularly applied to the elder users.

Thereby, for example, for the elderly who have lost their appetite, the tasting experience may be enriched with multisensory cues and sounds designed such as to enhance the eating or drinking experience. Chewing sounds may be augmented so as as to let the meal appear more fresh and/or crisp. In particular, by a suitable sound stimulus presented to the hearing device user, the perceived taste of food may be influenced (e.g. low pitch sounds relate to bitter, salty flavors. High pitch sounds relate to sweet, sour flavors). This may be determined so as to stimulate the consumption of certain types of aliments, which better suit the dietary requirements of the user.

To support the determination of a suitable output and/or to support the sensor-based analysis algorithm, the user may enter the kind of drink or food via the above-described user interface, or a barcode on the drink or food package may be scanned with the connected user device such as smartphone.

By the method described herein, for example, obesity and a risk of heart deseases due to bad eating (or also drinking) habits of the users may be effectively reduced.

Further aspects of the invention relate to a computer program for detecting and quantifying a liquid and/or food intake of a user wearing a hearing device which comprises at least one microphone, which program, when being executed by a processor, is adapted to carry out the steps of the method as described above and in the following as well as to a computer-readable medium, in which such a computer program is stored.

For example, the computer program may be executed in a processor of a hearing device, which hearing device, for example, may be carried by the person behind the ear. The computer-readable medium may be a memory of this hearing device. The computer program also may be executed by a processor of a connected user device, such as a smartphone or any other type of mobile device, which may be a part of the hearing system, and the computer-readable medium may be a memory of the connected user device. It also may be that some steps of the method are performed by the hearing device and other steps of the method are performed by the connected user device.

In general, a computer-readable medium may be a floppy disk, a hard disk, an USB (Universal Serial Bus) storage device, a RAM (Random Access Memory), a ROM (Read Only Memory), an EPROM (Erasable Programmable Read Only Memory) or a FLASH memory. A computer-readable medium may also be a data communication network, e.g. the Internet, which allows downloading a program code. The computer-readable medium may be a non-transitory or transitory medium.

Further aspects of the invention relate to a hearing device worn by a hearing device user and to a hearing system comprising such a hearing device, as described herein above and below. The hearing device or, respectively, the hearing system is adapted for performing the method described herein above and below. As already mentioned further above, the hearing system may further include, by way of example, a second hearing device worn by the same user and/or a connected user device, such as a smartphone or other mobile device, or personal computer, used by the same user.

According to an embodiment of the invention, the hearing device comprises: a microphone; a processor for processing a signal from the microphone; a sound output device for outputting the processed signal to an ear of the hearing device user; and, as the case may be, a transceiver for exchanging data with the connected user device and/or with another hearing device worn by the same user.

It has to be understood that features of the method as described above and in the following may be features of the computer program, the computer-readable medium and the hearing device and the hearing system as described above and in the following, and vice versa.

These and other aspects of the invention will be apparent from and elucidated with reference to the embodiments described hereinafter.

### BRIEF DESCRIPTION OF THE DRAWINGS

Below, embodiments of the present invention are described in more detail with reference to the attached drawings.
Fig. 1 schematically shows a hearing system according to an embodiment of the invention.
Fig. 2 shows a flow diagram of a method according to an embodiment of the invention for detecting and quantifying a liquid and/or food intake of a user wearing a hearing device of the hearing system of Fig. 1.

The reference symbols used in the drawings, and their meanings, are listed in summary form in the list of reference symbols.

### DETAILED DESCRIPTION OF EXEMPLARY EMBODIMENTS

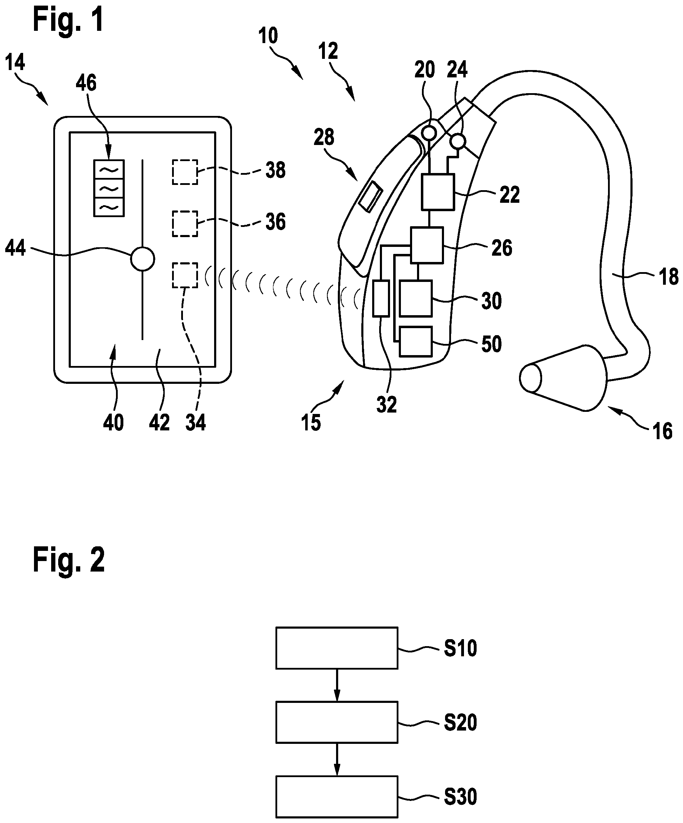

Fig. 1 schematically shows a hearing system 10 including a hearing device 12 in the form of a behind-the-ear device carried by a hearing device user (not shown) and a connected user device 14, such as a smartphone or a tablet computer. It has to be noted that the hearing device 12 is a specific embodiment and that the method described herein also may be performed with other types of hearing devices, such as in-the-ear devices.

The hearing device 12 comprises a part 15 behind the ear and a part 16 to be put in the ear canal of the user. The part 15 and the part 16 are connected by a tube 18. In the part 15, at least one microphone 20, a sound processor 22 and a sound output device 24, such as a loudspeaker, are provided. The microphone(s) 20 may acquire environmental sound of the user and may generate a sound signal, the sound processor 22 may amplify the sound signal and the sound output device 24 may generate sound that is guided through the tube 18 and the in-the-ear part 16 into the ear canal of the user.

The hearing device 12 may comprise a processor 26 which is adapted for adjusting parameters of the sound processor 22 such that an output volume of the sound signal is adjusted based on an input volume. These parameters may be determined by a computer program run in the processor 26. For example, with a knob 28 of the hearing device 12, a user may select a modifier (such as bass, treble, noise suppression, dynamic volume, etc.) and levels and/or values of these modifiers may be selected, from this modifier, an adjustment command may be created and processed as described above and below. In particular, processing parameters may be determined based on the adjustment command and based on this, for example, the frequency dependent gain and the dynamic volume of the sound processor 22 may be changed. All these functions may be implemented as computer programs stored in a memory 30 of the hearing device 12, which computer programs may be executed by the processor 26 and/or sound processor 22.

The hearing device 12 further comprises a transceiver 32 which may be adapted for wireless data communication with a transceiver 34 of the connected user device 14, which may be a smartphone or tablet computer. It is also possible that the above-mentioned modifiers and their levels and/or values are adjusted with the connected user device 14 and/or that the adjustment command is generated with the connected user device 14. This may be performed with a computer program run in a processor 36 of the connected user device 14 and stored in a memory 38 of the connected user device 14. The computer program may provide a graphical user interface 40 on a display 42 of the connected user device 14.

For example, for adjusting the modifier, such as volume, the graphical user interface 40 may comprise a control element 44, such as a slider. When the user adjusts the slider, an adjustment command may be generated, which will change the sound processing of the hearing device 12 as described above and below. Alternatively or additionally, the user may adjust the modifier with the hearing device 12 itself, for example via the knob 28.

The user interface 40 also may comprise an indicator element 46, which, for example, displays a currently determined listening situation.

The hearing device 12 further comprises at least one further sensor 50, the position of which in the part 15 of the hearing device 12 is to be understood as only symbolical, i.e. the further sensors 50 may also be provided in other parts and at other positions in the hearing device 12. The microphone(s) 20 and the further sensor(s) 50 enable the hearing device 12 or the hearing system 10 to perform a multi-sensor-based analysis for detecting and quantifying a liquid and/or food intake of a user as described herein. The further sensor(s) 50 may be integrated not only in the hearing device 12, but additionally also in the connected user devices such as the connected user device 14 shown in Fig. 1, or a wearable such as a wrist band etc. (not shown). As described in more detail herein above and below, the further sensors 50 may include any types of physical or physiological sensors - e.g. movement sensors, such as accelerometers, and/or optical sensors, such as cameras, and/or temperature sensors and/or heart rate sensors etc.

The hearing system 10 shown in Fig. 1 is adapted for performing a method according to the present invention for detecting and quantifying a liquid and/or food intake of a user wearing the hearing device 12, as described in more detail herein above.

Fig. 2 shows an example for a flow diagram of this method according to an embodiment of the invention. The method may be a computer-implemented method performed automatically in the hearing system 10 of Fig. 1.

In a first step S10 of the method, an audio signal from the at least one microphone 20 and/or a sensor signal from the at least one further sensor 50 is received, e.g. by the sound processor 22 and the processor 26 of the hearing device 12.

In a second step S20 of the method, the signal(s) received in step S10 are collected and analyzed in the hearing device 12 or in the hearing system 10, so as to detect each time the user drinks and/or eats something, wherein drinking is distinguished from eating, and so as to determine values indicative of how often this is detected and/or a respective amount of liquid and/or food ingested by the user. The step of analyzing includes applying one or more machine learning algorithms, which may be implemented on a processor chip integrated in the hearing device 12, or alternatively in the connected user device 14 or somewhere else in the hearing system or in a remote server or cloud connected to it.

In a third step S30 of the method, the determined values are stored in the hearing system 10 and, based on the stored values, a predetermined type of output as described in more detail herein above and below is generated, for example, to the user and/or to a third party, such as a person close to the user and/or a health care professional.

In the following, the method of the present invention will be illustrated, by way of example only, for estimating a risk of dehydration of the user and generating a corresponding output to counsel the user to drink more, and for verifying a medication intake based on the detected liquid intake. It may also be applied in a similar manner for other use cases described in detail herein above and in the claims, such as enhancing the user's desire to drink or enhancing the eating or drinking experience of the user.

In this example, the solution according to the present method comprises a so-called "sensory part", which may cover the above step S10 and at least a part of step S20 and comprise detecting how many times and how long the user is drinking. It further comprises a so-called "actuator part", which may cover the above step S30 and at least a part of step S20 and comprise storing the detected values into the memory of the hearing device or into the memory of an additional device such as a smartwatch, a wristband, a cloud etc.; estimating the risk of dehydration and counselling the user and/or, if the user agrees, informing a third party about his drinking behavior, such as a doctor, nurses or caregivers. That is, if the user agrees, his data and values determined in the analysis step of the method are sent to third parties (in addition or alternatively to counseling the user).

The sensory part is capable to detect how often and how many swallows or gulps the user takes each time. It is also capable to distinguish between drinking and eating, own-voice and other possible confusions. Three (to four) phases of drinking might be distinguished, such as:
Phase 1: Grabbing a glass or bottle and bring it to the mouth
Phase 2: Gulping the liquid
Phase 3: Putting the glass back on the table. This may also include detecting the sub-phases of (3a) a movement to bring the glass/bottle from the mouth down to the table and (3b) the actual putting of the glass on the table.

Different sensors might be better suited to detect the different phases, for example:
Phase 1: movement sensors within a wrist band and a hearing device might be most suited,
Phase 2: microphones within the hearing device and accelerometers within the ear canal might be most suited,
Phase 3: movement sensors on wrist band and microphones might be most suited.

The hearing device 12 or system 10 may combine and analyse signals of several sensors to detect all phases of the drinking procedure:
- Accelerometers (or gyroscopes) in the hearing device may be used to detect when the user elevates his head slightly to take a sip. In contrast, when eating, the user is rather lowering his head when taking a bite.
- In addition, the user might wear a smartwatch or a wristband around his wrist with integrated movement sensors. While the user brings the glass to his mouth and while drinking, his arm follows a specific gesture, which indicates the possible action of drinking. This movement pattern might also be used to distinguish between drinking and other possible actions of the user.
- In addition, a drinking bottle might incorporate sensors and be connected to the smart home (also known as smart water bottles). The hearing device 12 or system 10, also connected to the smart home, receives those signals to incorporate them into the detection process. Alternatively, those data are collected in a cloud or a smart home device to compute the algorithms.
- Heart rate sensors might be incorporated to detect fluid intake: the pulse of the heart can be influenced and altered by swallowing liquid or solid nutrition.
- Other sensors, such as electroencephalographic (EEG) sensors, eye gaze or a camera within the smart home or integrated into glasses might be incorporated for detection.

In addition, other sensors might be applied, such as:
- Acoustic sensors: the microphones within the hearing device or microphones in the ear canal might detect the gulping sound or might improve the detection rate by excluding other actions, such as own-voice or eating.
- Voice Pickup sensors (VPUs) might be used to improve the detection of gulping and distinguish it from eating.

The outcome of the several sensors is fed into a detection algorithm. The detection algorithm might consist of three parts, which are processed in a timely sequence, according to the three drinking phases described above. The algorithms might be a (deep) neural network, multivariate analysis of variance (Manova), Support Vector Machines (SVM) or any other machine learning algorithm, pattern recognition algorithm or statistical approach.

Instead of processing the three phases in a temporal manner and separately, the drinking procedure might be also detected without distinguishing any phases. A deep learning algorithm might find other criteria during a training phase. Since dehydration and drinking detection must not be solved in real time and must not be processed within the hearing device 12, diverse and more complex algorithms and processes are applicable.

Description of the method based on (deep) neural networks:
∘ Input: data sets x = (t, x1, x2, x3, ...) for each time instance (t), where x1, x2, x3,... represent the collected sensor values, as described above.
∘ Output data: y = (t, fi, d) for the input time instance (t), where (fi) is the determined number and (d) the determined duration of fluid intake in this example.
∘ Supervised learning: the algorithm is trained with labeled data.

The learning part can be supervised and trained with a database of sensor data. Here, a suitable database of training data based on the audio signal from a microphone contains recorded swallowing or gulping sound data of a large number of people - in this example 1000 randomly selected persons. Alternatively or additionally, representative sound data of drinking, swallowing, gulping, chewing etc. available in the Internet may be used as training data. The training can also be unsupervised by learning in an environment with more information available, such as using smart water bottles and/or information within a smart home and/or input from the user. Reinforcement learning or "deep reinforcement learning", as a third category of machine learning paradigms beside supervised and unsupervised learning, proposes different algorithms, which may be applied in the present method as well.

Instead of distinguishing three phases of drinking a priori, the "history" of the drinking process or, respectively, its temporal dynamic behaviour, might be incorporated by applying different machine learning methods. Beside the traditional method of Hidden Markow Model (HMM), recurrent neural networks (RNN) might be applied, e.g.
▪ Long short-term memory (LSTM)
▪ Gated recurrent unit (GRU).

GRU might be more suited to the application due to less computational effort.

Furthermore, the analysis may be implemented by parallel decision units in the hearing device 12 or in the hearing system 10. That is, instead of using one algorithm, several machine learning (ML) algorithms might be applied in parallel to compute the detection probabilities.

This may, for example, allow for the following features:
▪ To detect some drinking phases, one or the other sensor and/or algorithm might be more suitable than the other.
▪ Weighting parameters (e.g. in the activation functions of the artificial neurons) might be separately introduced and determined for each drinking phase and each decision algorithm.
▪ An algorithm in sequence to the several decision algorithms, e.g. in an architecture such as a decision tree, might be used to make the final decision.

As already mentioned, the sensor data might be collected and analysed in different types of analysis units, for example:
- within the hearing device 12, the hearing system 10 incorporating additional devices such as smartphone 14, smartwatch, wrist band, remote control, remote microphone, hearing aid/device etui, TV-Connector, ...
- on a server, cloud, smart home device.

As already mentioned above, following on the above-described "sensor part", the "actuator part" of the method consists of several steps in this example: storing the detected values in a memory, estimation of a dehydration risk, counselling the user, informing third party about drinking behaviour of the user. Two of these steps are further described in the following:
The risk of dehydration is estimated dependent on the detected drinking behavior, e.g. the determined amount of liquid ingested by the user is compared with a certain value "Dmin" describing the necessary minimum amount of drinking. This value can be a fixed number or might be calculated depending on one or more of the following factors:
- on the environmental condition, such as the weather temperature: if the temperature around the user is very high, the value "Dmin" is increased;
- on the activity behavior of the user: if he walks a lot (e.g. as detected by an accelerometer) or performs an activity with high physical effort (the heart beat is increased, e.g. as measured by a corresponding sensor), the value "Dmin" increases
- on medical conditions (e.g. as preset by the user's physician)

The values "Dmin" might be stored in a table including predetermined values or calculated with a regression equation, e.g. as known from medical studies. Alternatively or in addition, the value "Dmin" can also be adapted by the user or a doctor.

The above-mentioned step of counseling the user might include several options, such as one or more of the following:
- a smartphone app on the connected user device 14 displays how much the user has drunk and how much the user should have drunk during the day.
- the user gets notified when he shall drink: acoustically, via vibration on devices such as wristband, smartwatch, or via augmented reality. This may, for example, be implemented in that the user's glasses (or smart glasses being a part of the hearing system 10 or connected to it) augment visually a drinking glass or a drinking bottle.

Moreover, in the present example, an interactive user interface is provided in the hearing system 10 and the steps of analyzing (S20) and/or generating an output (S30) are supplemented by an interaction with the user via this user interface, wherein the user is enabled to input additional information pertaining to his liquid intake.

For example, such an interface may be configured such as to enable the user to manually enter the data for drinking behaviour and/or to correct and/or specify the liquid intake. It might further provide the user with a possibility to add comments about subjective descriptions and ratings about his health state, mental condition, feelings, thirst, appetite, social condition (e.g. alone or with company), cause of fluid intake, intake of medication, etc.

Thereby, for example, the hearing system 10 might ask the user proactively whether he has taken medication right after the system has detected fluid intake in step S20. The proactive questioning might be during a specific time predefined and stored in the hearing system 10, such as in the morning, in the evening, just before or after taking a meal, which, in turn, might be determined using the analysis algorithm in step S20. This proactive question shall help the user to keep track about the intake of medication.

The interactive user interface might also help to improve the accuracy of the detection in general or within a learning phase of the analysis algorithm in step S20. It may also serve for a more elaborated therapy.

The interactive user interface (also denoted as Ul) might comprise one or more of the following types of user interface:
- an app on a smartphone (connected user device 14 in Fig. 1), smartwatch or another connected user device, which provides a correction mode showing e.g. the detected gulpes right after detection (e.g. the graphical user interface 40 on the display 42 of the connected user device 14).
- An entry mode provided in the hearing device (e.g. via the slider 44 in the graphical user interface 40 on the connected user device 14), configured to enable the user to specify the liquid intake.
- A conversational user interface incorporated in the hearing device 12.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. A single processor or controller or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SYMBOLS

- 10: hearing system
- 12: hearing device
- 14: connected user device
- 15: part behind the ear
- 16: part in the ear
- 18: tube
- 20: microphone(s)
- 22: sound processor
- 24: sound output device
- 26: processor
- 28: knob
- 30: memory
- 32: transceiver
- 34: transceiver
- 36: processor
- 38: memory
- 40: graphical user interface, interactive user interface
- 42: display
- 44: control element, slider
- 46: indicator element
- 50: further sensor, in particular a movement sensor, such as an accelerometer

## Claims

1. A method for detecting and quantifying a liquid and/or food intake of a user wearing a hearing device (12) which comprises at least one microphone (20), the method comprising:
receiving an audio signal from the at least one microphone (20) and/or a sensor signal from at least one further sensor (50);
collecting and analyzing the received audio signal and/or further sensor signals so as to detect each time the user drinks and/or eats something, wherein drinking is distinguished from eating, and so as to determine values indicative of how often this is detected and/or a respective amount of liquid and/or food ingested by the user;
wherein the step of analyzing includes applying one or more machine learning algorithms in the hearing device (12) or in a hearing system (10), part of which the hearing device (12) is, or in a remote server or cloud connected to it; and
storing the determined values in the hearing system (10) and, based on the stored values, generating a predetermined type of output.

2. The method of claim 1, wherein, in the step of analyzing,
at least one of the machine learning algorithms is applied in its training phase so as to learn user-specific manners of drinking and/or eating; and
the newly learned user-specific manners are incorporated in the future analysis step.

3. The method of claim 1 or 2, wherein, in the step of analyzing,
two or more different phases of drinking or, respectively, eating, are distinguished in the course of detecting a liquid and/or food intake of the user; and
the analysis of the different phases is based on signals from correspondingly different sensors (20, 50) and/or is performed by correspondingly different machine learning algorithms.

4. The method of claim 3, wherein the different phases of drinking comprise one or more of the following phases:
bringing a source of liquid in contact with the mouth, based at least on a signal from at least one movement sensor (50) sensing a corresponding movement of some upper body part of the user;
gulping or sipping the liquid, based at least on a signal from the at least one microphone (20) and/or on a signal from at least one movement sensor (50) sensing a corresponding movement of the user's throat, head and/or breast;
removing the mouth from the source of liquid, based at least on a signal from the at least one microphone (20) and/or on a signal from at least one movement sensor (50) sensing a corresponding movement of some upper body part of the user.

5. The method of one of the previous claims, wherein
at least one of the machine learning algorithms is based on an artificial neural network;
the input data set for the neural network is provided at a respective time point by the sensor data collected over a predetermined period of time up to this time point;
the output data set for the respective time point includes a frequency or number of detected liquid and/or food intakes as well as a respective or an overall amount of the liquid and/or food ingested by the user and/or a duration of the detected liquid and/or food intakes;
wherein the learning phase is implemented by a supervised learning, in which the algorithm is trained using a database of input sensor data with labeled output data sets; or, alternatively, by an unsupervised learning in an environment with more information available and/or by a reinforcement learning or deep reinforcement learning.

6. The method of claim 5, wherein
the artificial neural network is a deep neural network including at least one hidden layer.

7. The method of one of the previous claims, wherein, in the step of analyzing,
a temporal dynamic behavior of the drinking and/or eating process, is incorporated by applying at least one of the following machine learning methods:
a Hidden Markow Model;
a recurrent neural network.

8. The method of one of the previous claims, wherein,
in the step of analyzing, a dehydration risk of the hearing device user is estimated depending on the determined values of the amount and of a frequency of the user's liquid intake; and
the generated output is configured depending on the estimated dehydration risk, so as to counsel the user to ingest a lacking amount of liquid and/or so as to inform the user and/or a person close to the user and/or a health care professional about the estimated dehydration risk.

9. The method of one of the previous claims, wherein
an interactive user interface (40) is provided in the hearing system (10); and
the steps of analyzing and/or generating an output are supplemented by an interaction with the user via the interactive user interface (40), wherein the user is enabled to input additional information pertaining to his liquid and/or food intake.

10. The method of claim 9, wherein
additional information about a need to take a predetermined medication is stored in the hearing system (10);
when a fluid intake of the user is detected, the output is generated depending on this additional information and comprises questioning the user, via the interactive user interface (40), whether he has taken the predetermined medication;
and the user's response to this question via the interactive user interface (40) is stored in the hearing system (10) and/or transmitted to the user and/or a person close to the user and/or a health care professional, so as to verify that the user has taken the predetermined medication.

11. The method of one of the previous claims, wherein, in the step of generating an output,
depending on the determined frequency and amount of the liquid or, respectively, food ingested by the user, an output configured such as to enhance the user's desire to drink or, respectively, to eat something is generated by augmented reality means in the hearing system (10).

12. The method of one of the previous claims, wherein
when detecting that the user is drinking or, respectively, eating something and/or upon detecting which kind of liquid or, respectively, food the user is taking, an output configured such as to enhance the user's experience of drinking or, respectively, eating is generated by augmented reality means in the hearing system (10) depending on this detection.

13. A computer program for detecting and quantifying a liquid and/or food intake of a user wearing a hearing device (12) which comprises at least one microphone (20), which program, when being executed by a processor (26, 36), is adapted to carry out the steps of the method of one of the previous claims.

14. A computer-readable medium, in which a computer program according to claim 13 is stored.

15. A hearing device (12) worn by a hearing device user, comprising:
a microphone (20);
a processor (26) for processing a signal from the microphone (20);
a sound output device (24) for outputting the processed signal to an ear of the hearing device user;
wherein the hearing device (12) is adapted for performing the method of one of claims 1 to 12.

16. A hearing system (10) comprising a hearing device (12) worn by a hearing device user and a connected user device (14) and/or a second hearing device worn by the same user, wherein the hearing device (12) comprises:
a microphone (20);
a processor (26) for processing a signal from the microphone (20);
a sound output device (24) for outputting the processed signal to an ear of the hearing device user; and
a transceiver (32) for exchanging data with the connected user device (14) and/or with the second hearing device;
wherein the hearing system (10) is adapted for performing the method of one of claims 1 to 12.
